# EUROPEAN PATENT SPECIFICATION

(11) **EP 3 367 830 B1**
(45) Date of publication and mention of the grant of the patent: **07.10.2020**
(21) Application number: 16798651.2
(22) Date of filing: 26.10.2016
(51) Int. Cl.: A24F 47/00

(54) **ARTICLE FOR USE WITH APPARATUS FOR HEATING SMOKABLE MATERIAL**
ARTIKEL ZUR VERWENDUNG MIT EINER VORRICHTUNG ZUR ERWÄRMUNG VON RAUCHBAREM MATERIAL
ARTICLE À UTILISER AVEC UN APPAREIL POUR CHAUFFER UNE SUBSTANCE À FUMER

(30) Priority: 30.10.2015 US 201514927532
(43) Date of publication of application: 05.09.2018
(73) Proprietor: British American Tobacco (Investments) Limited, London WC2R 3LA (GB)
(72) Inventor: BLANDINO, Thomas, P., Cottage Grove, Wisconsin 53527 (US); WILKE, Andrew, P., Madison, Wisconsin 53704 (US); FRATER, James, J., Madison, Wisconsin 53718 (US); PAPROCKI, Benjamin, J., Cottage Grove, Wisconsin 53527 (US)
(74) Representative: EIP
(86) International application number: PCT/EP2016/075739
(87) International publication number: WO 2017/072149

(56) References cited:
- WO-A1-95/27411
- WO-A1-2014/048745
- US-A- 4 987 291
- US-A- 5 272 216
- US-A1- 2015 245 669
- TODAKA T ET AL: "Low Curie temperature material for induction heating self-temperature controlling system", JOURNAL OF MAGNETISM AND MAGNETIC MATERIALS, ELSEVIER, AMSTERDAM, NL, vol. 320, no. 20, 1 October 2008 (2008-10-01), pages e702-e707, XP023905279, ISSN: 0304-8853, DOI: 10.1016/J.JMMM.2008.04.146 [retrieved on 2008-04-29] cited in the application

## Description

### Technical Field

The present invention relates to apparatus for heating smokable material to volatilise at least one component of the smokable material, and to methods of manufacturing products comprising heaters for use in heating smokable material to volatilise at least one component of the smokable material.

### Background

Smoking articles such as cigarettes, cigars and the like burn tobacco during use to create tobacco smoke. Attempts have been made to provide alternatives to these articles by creating products that release compounds without combusting. Examples of such products are so-called "heat not burn" products or tobacco heating devices or products, which release compounds by heating, but not burning, material. The material may be, for example, tobacco or other non-tobacco products, which may or may not contain nicotine.

WO95/27411 discloses an induction heating source for use with a smoking article. The induction heating source provides an alternating electromagnetic field which inductively heats a susceptor in thermal proximity with tobacco flavor medium to generate aerosols.

### Summary

A first aspect of the present invention provides a method of manufacturing a product comprising a heater for use in heating smokable material to volatilise at least one component of the smokable material, the method being as defined in claim 1.

In an exemplary embodiment, the Curie point temperature is equal to or less than the maximum temperature.

In an exemplary embodiment, the maximum temperature is less than the combustion temperature of the smokable material to be heated by the heater in use.

In an exemplary embodiment, the combustion temperature of the smokable material is the autoignition temperature or kindling point of the smokable material.

In an exemplary embodiment, the Curie point temperature is no more than 350 degrees Celsius.

In respective exemplary embodiments, the Curie point temperature may be less than 350 degrees Celsius, less than 325 degrees Celsius, less than 300 degrees Celsius, less than 280 degrees Celsius, less than 260 degrees Celsius, less than 240 degrees Celsius, or less than 220 degrees Celsius.

In an exemplary embodiment, the heating material comprises one or more materials selected from the group consisting of: iron; an alloy comprising iron; an alloy comprising iron and nickel; an alloy comprising iron and nickel and chromium; an alloy comprising iron and nickel and chromium and manganese; an alloy comprising iron and nickel and chromium and manganese and silicon; and stainless steel.

In an exemplary embodiment, the heater consists entirely, or substantially entirely, of the heating material.

A second aspect of the present invention provides apparatus for heating smokable material to volatilise at least one component of the smokable material, the apparatus comprising:
a heating zone for receiving an article comprising smokable material;
a heater for heating the heating zone, wherein the heater comprises heating material that is heatable by penetration with a varying magnetic field; and
a magnetic field generator for generating a varying magnetic field that penetrates the heating material;
wherein a maximum temperature to which the heater is heatable by penetration with the varying magnetic field in use is exclusively determined by a Curie point temperature of the heating material.

In an exemplary embodiment, the Curie point temperature is no more than 350 degrees Celsius.

In respective exemplary embodiments, the Curie point temperature may be less than 350 degrees Celsius, less than 325 degrees Celsius, less than 300 degrees Celsius, less than 280 degrees Celsius, less than 260 degrees Celsius, less than 240 degrees Celsius, or less than 220 degrees Celsius.

In an exemplary embodiment, the heating material comprises one or more materials selected from the group consisting of: iron; an alloy comprising iron; an alloy comprising iron and nickel; an alloy comprising iron and nickel and chromium; an alloy comprising iron and nickel and chromium and manganese; an alloy comprising iron and nickel and chromium and manganese and silicon; and stainless steel.

In an exemplary embodiment, the heater consists entirely, or substantially entirely, of the heating material.

A third aspect of the present invention provides a system, comprising:
apparatus for heating smokable material to volatilise at least one component of the smokable material; and
an article for use with the apparatus, wherein the article comprises smokable material;
wherein the apparatus comprises:
   a heating zone for receiving the article,
   a heater for heating the smokable material when the article is in the heating zone, wherein the heater is formed of heating material that is heatable by penetration with a varying magnetic field, and
   a magnetic field generator for generating a varying magnetic field that penetrates the heating material;
wherein a maximum temperature to which the heater is heatable by penetration with the varying magnetic field in use is exclusively determined by a Curie point temperature of the heating material.

### Brief Description of the Drawings

Embodiments of the invention will now be described, by way of example only, with reference to the accompanying drawings, in which:
Figure 1 shows a schematic perspective view of an example of an article for use with apparatus for heating smokable material to volatilise at least one component of the smokable material;
Figure 2 shows a schematic cross-sectional view of the article of Figure 1;
Figure 3 shows a schematic cross-sectional view of an example of apparatus for heating smokable material to volatilise at least one component of the smokable material;
Figure 4 is a flow diagram showing an example of a method of manufacturing an article for use with apparatus for heating smokable material to volatilise at least one component of the smokable material; and
Figure 5 is a flow diagram showing an example of a method of manufacturing apparatus for heating smokable material to volatilise at least one component of the smokable material.

### Detailed Description

As used herein, the term "smokable material" includes materials that provide volatilised components upon heating, typically in the form of vapour or an aerosol. "Smokable material" may be a non-tobacco-containing material or a tobacco-containing material. "Smokable material" may, for example, include one or more of tobacco per se, tobacco derivatives, expanded tobacco, reconstituted tobacco, tobacco extract, homogenised tobacco or tobacco substitutes. The smokable material can be in the form of ground tobacco, cut rag tobacco, extruded tobacco, reconstituted tobacco, reconstituted smokable material, liquid, gel, gelled sheet, powder, or agglomerates, or the like. "Smokable material" also may include other, non-tobacco, products, which, depending on the product, may or may not contain nicotine. "Smokable material" may comprise one or more humectants, such as glycerol or propylene glycol.

As used herein, the term "heating material" or "heater material" refers to material that is heatable by penetration with a varying magnetic field.

Induction heating is a process in which an electrically-conductive object is heated by penetrating the object with a varying magnetic field. The process is described by Faraday's law of induction and Ohm's law. An induction heater may comprise an electromagnet and a device for passing a varying electrical current, such as an alternating current, through the electromagnet. When the electromagnet and the object to be heated are suitably relatively positioned so that the resultant varying magnetic field produced by the electromagnet penetrates the object, one or more eddy currents are generated inside the object. The object has a resistance to the flow of electrical currents. Therefore, when such eddy currents are generated in the object, their flow against the electrical resistance of the object causes the object to be heated. This process is called Joule, ohmic, or resistive heating. An object that is capable of being inductively heated is known as a susceptor.

It has been found that, when the susceptor is in the form of a closed circuit, magnetic coupling between the susceptor and the electromagnet in use is enhanced, which results in greater or improved Joule heating.

Magnetic hysteresis heating is a process in which an object made of a magnetic material is heated by penetrating the object with a varying magnetic field. A magnetic material can be considered to comprise many atomic-scale magnets, or magnetic dipoles. When a magnetic field penetrates such material, the magnetic dipoles align with the magnetic field. Therefore, when a varying magnetic field, such as an alternating magnetic field, for example as produced by an electromagnet, penetrates the magnetic material, the orientation of the magnetic dipoles changes with the varying applied magnetic field. Such magnetic dipole reorientation causes heat to be generated in the magnetic material.

When an object is both electrically-conductive and magnetic, penetrating the object with a varying magnetic field can cause both Joule heating and magnetic hysteresis heating in the object. Moreover, the use of magnetic material can strengthen the magnetic field, which can intensify the Joule heating.

In each of the above processes, as heat is generated inside the object itself, rather than by an external heat source by heat conduction, a rapid temperature rise in the object and more uniform heat distribution can be achieved, particularly through selection of suitable object material and geometry, and suitable varying magnetic field magnitude and orientation relative to the object. Moreover, as induction heating and magnetic hysteresis heating do not require a physical connection to be provided between the source of the varying magnetic field and the object, design freedom and control over the heating profile may be greater, and cost may be lower.

The Curie point temperature, or Curie Temperature, is the temperature at which certain magnetic materials undergo a sharp change in their magnetic properties. It is understood that the Curie point temperature is the temperature below which there is spontaneous magnetisation in the absence of an externally applied magnetic field, and above which the material is paramagnetic. For example, the Curie point temperature is the magnetic transformation temperature of a ferromagnetic material between its ferromagnetic and paramagnetic phase. When such a magnetic material reaches its Curie point temperature, its magnetic permeability reduces or ceases, and the ability of the material to be heated by penetration with a varying magnetic field also reduces or ceases. That is, it may not be possible to heat the material above its Curie point temperature by magnetic hysteresis heating. If the magnetic material is electrically-conductive, then the material may still be heatable, to a lesser extent, by penetration with a varying magnetic field above the Curie point temperature by Joule heating. However, if the magnetic material is non-electrically-conductive, then heating of the material above its Curie point temperature by penetration with a varying magnetic field may be hindered or even impossible.

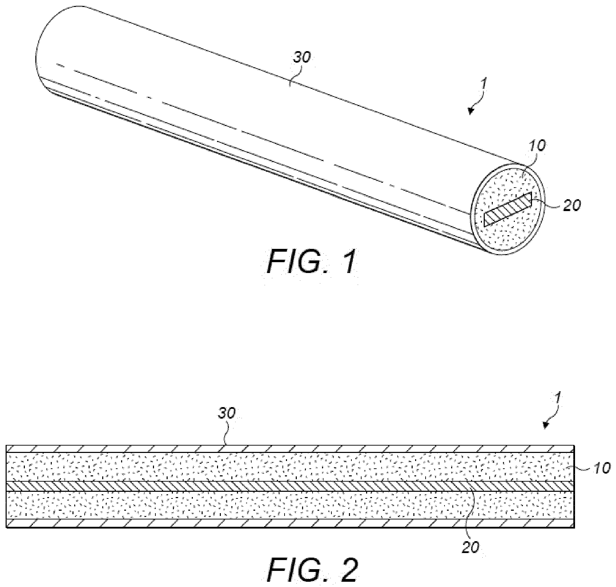

Referring to Figures 1 and 2 there are shown a schematic perspective view and a schematic cross-sectional view of an example of an article. Broadly speaking, the article 1 comprises smokable material 10, a heater 20 for heating the smokable material 10, and a cover 30 that encircles the smokable material 10 and the heater 20. The heater 20 comprises heating material that is heatable by penetration with a varying magnetic field. Example such heating materials are discussed elsewhere herein. The article 1 is for use with apparatus for heating the smokable material 10 to volatilise at least one component of the smokable material 10 without burning the smokable material 10.

The article 1 is elongate and cylindrical with a substantially circular cross section in a plane normal to a longitudinal axis of the article 1. However, in other constructions, the article 1 may have a cross section other than circular and/or not be elongate and/or not be cylindrical. The article 1 may have proportions approximating those of a cigarette.

The heater 20 is elongate and extends along a longitudinal axis that is substantially aligned with a longitudinal axis of the article 1. This can help to provide more uniform heating of the smokable material 10 in use, and can also aid manufacturing of the article 1. The aligned axes are coincident. In a variation to this construction, the aligned axes may be parallel to each other. However, in other constructions, the axes may be oblique to each other.

The heater 20 extends to opposite longitudinal ends of the mass of smokable material 10. This can help to provide more uniform heating of the smokable material 10 in use, and can also aid manufacturing of the article 1. However, in other constructions, the heater 20 may not extend to either of the opposite longitudinal ends of the mass of smokable material 10, or may extend to only one of the longitudinal ends of the mass of smokable material 10 and be spaced from the other of the longitudinal ends of the mass of smokable material 10.

The heater 20 is within the smokable material 10. In other constructions, the smokable material 10 may be on only one side of the heater 20, for example.

The heating material of the heater 20 is in contact with the smokable material 10. Thus, when the heating material is heated by penetration with a varying magnetic field, heat may be transferred directly from the heating material to the smokable material 10. In other constructions, the heating material may be kept out of contact with the smokable material 10. For example, in some constructions, the article 1 may comprise a thermally-conductive barrier that is free of heating material and that spaces the heater 20 from the smokable material 10. The thermally-conductive barrier may be a coating on the heater 20. The provision of such a barrier may be advantageous to help to dissipate heat to alleviate hot spots in the heating material.

The heater 20 has two opposing major surfaces joined by two minor surfaces. Therefore, the depth or thickness of the heater 20 is relatively small as compared to the other dimensions of the heater 20. The heating material may have a skin depth, which is an exterior zone within which most of an induced electrical current and/or induced reorientation of magnetic dipoles occurs. By providing that the heating material has a relatively small thickness, a greater proportion of the heating material may be heatable by a given varying magnetic field, as compared to heating material having a depth or thickness that is relatively large as compared to the other dimensions of the heating material. Thus, a more efficient use of material is achieved and, in turn, costs are reduced. However, in other constructions, the heater 20 may have a cross-section that is a shape other than rectangular, such as circular, elliptical, annular, polygonal, square, triangular, star-shaped, radially-finned, or the like.

The cover 30 of the article 1 helps to maintain the relative positions of the smokable material 10 and the heater 20. The cover 30 may be made of any suitable material, such as paper, card, a plastics material, or the like. Overlapping portions of the cover 30 may be adhered to each other to help maintain the shape of the cover 30 and the article 1 as a whole. In some constructions, the cover 30 may take a different form or be omitted.

The Curie point temperature of a material is determined or controlled by the chemical composition of the material. Modern technology allows adjustment of the composition of a material to provide the material with a preset Curie point temperature. Some example heating materials that could be used in embodiments of the present invention, along with their approximate Curie point temperatures, are as shown in Table 1, below.

**Table 1**

| **Material** | **Curie point temperature (degrees Celsius)** |
|---|---|
| 30% Ni 70% Fe | 100 |
| 36% Ni 64% Fe | 279 |
| 42% Ni 58% Fe | 325 |
| 46% Ni 54% Fe | 460 |
| 52% Ni 48% Fe | 565 |
| 80% Ni 20% Fe | 460 |
| Cobalt | 1120 |
| Iron | 770 |
| Low carbon steel | 760 |
| Iron (III) oxide | 675 |
| Iron (II, III) oxide | 585 |
| NiOFe₂O₃ | 585 |
| CuOFe₂O₃ | 455 |
| Strontium ferrite | 450 |
| MgOFe₂O₃ | 440 |
| Kovar * | 435 |
| MnBi | 357 |
| Nickel | 353 |
| MnSb | 314 |
| MnOFe₂O₃ | 300 |
| Y₃Fe₅O₁₂ | 287 |
| CrO₂ | 113 |
| MnAs | 45 |

| | |
|---|---|
| * A typical composition of Kovar is as follows, given in percentages of weight: Ni 29%, Co 17%, Si 0.2%, Mn 0.3%, C < 0.01%, Fe balance. | |

The % values given for the above various alloys of Ni and Fe may be %wt values.

*"*Low Curie temperature material for induction heating self-temperature controlling system"; T. Todaka et al.; Journal of Magnetism and Magnetic Materials 320 (2008) e702-e707, presents low Curie temperature magnetic materials for induction heating. The materials are alloys based on SUS430 (a grade of stainless steel), could be used in embodiments of the present invention, and are shown in Table 2, below, along with their approximate Curie point temperatures.

**Table 2**

| **Material Composition (wt %)** | **Curie point temperature (degrees Celsius)** |
|---|---|
| SUS430-Al_{11.7}Dy_{0.5} | 301 |
| SUS430-Al_{11.7}Gd_{0.3} | 300 |
| SUS430-Al_{11.7}Sm_{0.3} | 300 |
| SUS430-Al_{12.8}Gd_{0.3} | 194 |
| SUS430-Al_{12.8}Sm_{0.1} | 195 |
| SUS430-Al_{12.8}Y_{0.3} | 198 |
| SUS430-Al_{13.5}Gd_{0.3} | 106 |
| SUS430-Al_{13.5}Sm_{0.1} | 116 |
| SUS430-Al_{13.5}Y_{0.3} | 109 |

*"*Low Curie temperature in Fe-Cr-Ni-Mn alloys"; Alexandru Iorga et al.; U.P.B. Sci. Bull., Series B, Vol. 73, Iss. 4 (2011) 195-202, provides a discussion of several Fe-Ni-Cr alloys. Some of the materials disclosed in this document could be used in embodiments of the present invention, and are shown in Table 3, below, along with their approximate Curie point temperatures.

**Table 3**

| **Material Composition (wt %)** | **Curie point temperature (degrees Celsius)** |
|---|---|
| Cr₄-Ni₃₂-Fe₆₂-Mn_{1.5}-Si_{0.5} | 55 |
| Cr₄-Ni₃₃-Fe_{62.5}-Si_{0.5} | 122 |
| Cr₁₀-Ni33-Fe_{53.5}-Mn₃-Si_{0.5} | 11 |
| Cr₁₁-Ni₃₅-Fe_{53.5}-Si_{0.5} | 66 |

A further material that could be used in some embodiments of the present invention is NeoMax MS-135, which is from NeoMax Materials Co., Ltd.. This material is described at the following URL: http://www.neomax-materials.co.jp/eng/pr0510.htm.

In this construction, the chemical composition of the heating material of the heater 20 has been carefully and intentionally set, selected or provided so that the heating material has a Curie point temperature that is less than the combustion temperature of the smokable material 10. The combustion temperature may be the auto ignition temperature or kindling point of the smokable material 10. That is, the lowest temperature at which the smokable material 10 will spontaneously ignite in normal atmosphere without an external source of ignition, such as a flame or spark.

Accordingly, when the temperature of the heater 20 in use reaches the Curie point temperature, the ability to further heat the heater 20 by penetration with a varying magnetic field is reduced or removed. For example, as noted above, when the heating material is electrically-conductive, Joule heating may still be effected by penetrating the heating material with a varying magnetic field. Alternatively, when the heating material is non-electrically-conductive, depending on the chemical composition of the heating material, such further heating by penetration with a varying magnetic field may be impossible.

Thus, in use, this inherent mechanism of the heating material of the heater 20 may be used to limit or prevent further heating of the heater 20, so as to help avoid the temperature of the adjacent smokable material 10 from reaching a magnitude at which the smokable material 10 burns or combusts. Thus, the chemical composition of the heater 20 may help enable the smokable material 10 to be heated sufficiently to volatilise at least one component of the smokable material 10 without burning the smokable material 10. This may also help to prevent overheating of the apparatus with which the article 1 is being used, and/or help to prevent part(s), such as the cover 30 or an adhesive, of the article 1 being damaged by excessive heat during use of the article 1.

If the combustion temperature of the smokable material 10 is greater than X degrees Celsius, then the chemical composition of the heating material may be provided so that the Curie point temperature is no more than X degrees Celsius. For example, if the combustion temperature of the smokable material 10 is greater than 350 degrees Celsius, then the chemical composition of the heating material may be provided so that the Curie point temperature is no more than 350 degrees Celsius. The Curie point temperature may be, for example, less than 350 degrees Celsius, less than 325 degrees Celsius, less than 300 degrees Celsius, less than 280 degrees Celsius, less than 260 degrees Celsius, less than 240 degrees Celsius, or less than 220 degrees Celsius.

In some constructions, the ability of the heating material to be heated by penetration with a varying magnetic field by magnetic hysteresis heating may return when the temperature of the heating material has dropped below the Curie point temperature.

In some constructions, the heater 20 may consist entirely, or substantially entirely, of the heating material. The heating material may comprise, for example, one or more materials selected from the group consisting of: iron; an alloy comprising iron; an alloy comprising iron and nickel; an alloy comprising iron and nickel and chromium; an alloy comprising iron and nickel and chromium and manganese; an alloy comprising iron and nickel and chromium and manganese and silicon; and stainless steel.

In some embodiments, the heater of the product may comprise a first portion of heating material that has a first Curie point temperature, and a second portion of heating material that has a second Curie point temperature that is different to the first Curie point temperature. The second Curie point temperature may be higher than the first Curie point temperature. In use, the second portion of heating material may thus be permitted to reach a higher temperature than the first portion of heating material when both are penetrated by a varying magnetic field. This may help progressive heating of the smokable material 10, and thus progressive generation of vapour, to be achieved. Both the first and second Curie point temperatures may be less than the combustion temperature of the smokable material 10.

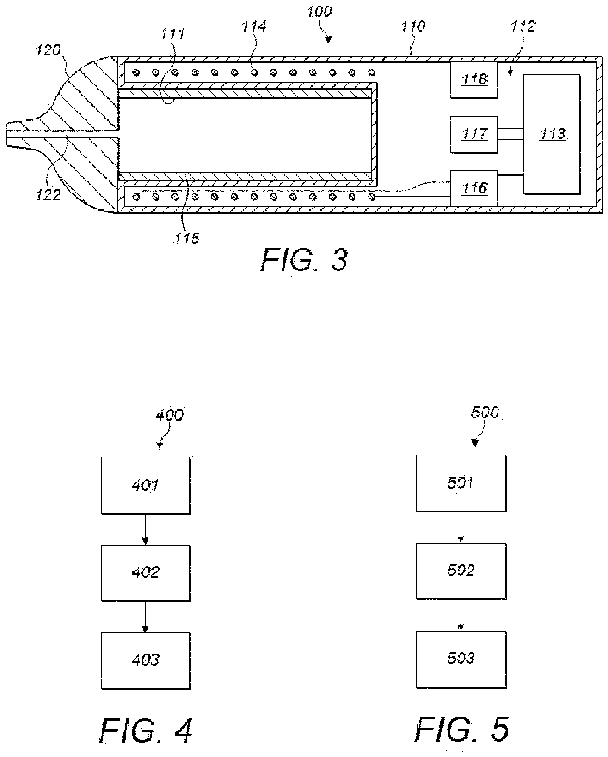

Referring to Figure 4, there is shown a flow diagram showing an example of a method of manufacturing a product for use in heating smokable material to volatilise at least one component of the smokable material. The article 1 of Figures 1 and 2 may be made according to this method.

The method 400 comprises determining 401 a maximum temperature to which a heater is to be heated in use. This determining 401 may comprise, for example, determining the combustion temperature of the smokable material 10 to be heated by the heater 20 in use, and then determining the maximum temperature on the basis of that combustion temperature. For example, the maximum temperature may be less than the combustion temperature of the smokable material 10, for the reasons discussed above. In other examples, the step of determining 401 may additionally or alternatively comprise determining a maximum temperature to which other part(s), such as a cover or an adhesive, of the article may be subjected in use without incurring damage, and then determining the maximum temperature on the basis of that temperature. For example, the maximum temperature may be less than the temperature to which the part(s) may be safely subjected in use. In still other examples, the step of determining 401 may additionally or alternatively comprise determining a maximum temperature to which the smokable material 10 is to be heated on the basis of desired sensory properties, and then determining the maximum temperature on the basis of that temperature. For example, at different temperatures different components of the smokable material 10 may be volatilised.

The method 400 further comprises providing 402 a heater 20 comprising heating material, wherein the heating material is heatable by penetration with a varying magnetic field, and wherein the heating material has a Curie point temperature selected or determined on the basis of, or in dependence on, the maximum temperature determined at 401. The step of providing 402 may comprise, for example, manufacturing the heater 20 from suitable heating material. The method may comprise adjusting the composition of the heating material during manufacture of the heater 20. Alternatively or additionally, the step of providing 402 may comprise selecting the heater 20 from a plurality of heaters 20, wherein the plurality of heaters 20 are made of heating material having respective different Curie point temperatures. The Curie point temperature of the heating material of the heater 20 provided in step 402 may, for example, be equal to the maximum temperature determined in step 401, or may be less than the maximum temperature determined in step 401. The heater 20 provided in step 402 may consists entirely, or substantially entirely, of the heating material. The heating material may comprise or consist of any one or more of the available heating materials discussed above, for example.

The method then comprises forming 403 an article, such as the article 1 of Figures 1 and 2, comprising the heater 20 and smokable material 10 to be heated by the heater 20 in use. The step of forming 403 may comprise providing that the heater 20 is in contact with the smokable material 10, as is the case in the article 1 of Figures 1 and 2. However, in other constructions, the smokable material 10 may be out of contact with the heater 20 and yet still be heatable by the heater 20. Step 403 of the method 400 may additionally or alternatively comprise encircling or covering the smokable material 10 and the heater 20 with a cover, such as the cover 30 of the article 1 shown in Figures 1 and 2.

The above-described article 1 and described variants thereof may be used with apparatus for heating the smokable material 10 to volatilise at least one component of the smokable material 10 without burning the smokable material 10. Any one of the article(s) 1 and such apparatus may be provided together as a system. The system may take the form of a kit, in which the article 1 is separate from the apparatus. Alternatively, the system may take the form of an assembly, in which the article 1 is combined with the apparatus. The apparatus of the system comprises a heating zone for receiving the article 1, and a magnetic field generator for generating a varying magnetic field that penetrates the heating material when the article 1 is in the heating zone.

Referring to Figure 3 there is shown a schematic cross-sectional view of an example of apparatus for heating smokable material to volatilise at least one component of the smokable material according to an embodiment of the invention. Broadly speaking, the apparatus 100 comprises a heating zone 111 for receiving an article comprising smokable material; a heater 115 for heating the heating zone 111, wherein the heater 115 comprises heating material that is heatable by penetration with a varying magnetic field; and a magnetic field generator 112 for generating a varying magnetic field that penetrates the heating material of the heater 115. A maximum temperature to which the heater 115 is heatable by penetration with the varying magnetic field in use is exclusively determined by a Curie point temperature of the heating material of the heater 115. Example such heating materials are discussed elsewhere herein. The apparatus 100 is for use with an article that comprises smokable material. In some embodiments, the apparatus 100 is for heating the smokable material to volatilise at least one component of the smokable material without burning the smokable material. The article may comprise heating material, such as the article 1 of Figures 1 and 2, or may be free of heating material.

The apparatus 100 of this embodiment comprises a body 110 and a mouthpiece 120. The mouthpiece 120 may be made of any suitable material, such as a plastics material, cardboard, cellulose acetate, paper, metal, glass, ceramic, or rubber. The mouthpiece 120 defines a channel 122 therethrough. The mouthpiece 120 is locatable relative to the body 110 so as to cover an opening into the heating zone 111. When the mouthpiece 120 is so located relative to the body 110, the channel 122 of the mouthpiece 120 is in fluid communication with the heating zone 111. In use, the channel 122 acts as a passageway for permitting volatilised material to pass from an article inserted in the heating zone 111 to an exterior of the apparatus 100. In this embodiment, the mouthpiece 120 of the apparatus 100 is releasably engageable with the body 110 so as to connect the mouthpiece 120 to the body 110. In other embodiments, the mouthpiece 120 and the body 110 may be permanently connected, such as through a hinge or flexible member. In some embodiments, such as embodiments in which the article itself comprises a mouthpiece, the mouthpiece 120 of the apparatus 100 may be omitted.

The apparatus 100 may define an air inlet that fluidly connects the heating zone 111 with the exterior of the apparatus 100. Such an air inlet may be defined by the body 110 of the apparatus 100 and/or by the mouthpiece 120 of the apparatus 100. A user may be able to inhale the volatilised component(s) of the smokable material by drawing the volatilised component(s) through the channel 122 of the mouthpiece 120. As the volatilised component(s) are removed from the article, air may be drawn into the heating zone 111 via the air inlet of the apparatus 100.

In this embodiment, the body 110 comprises the heating zone 111. In this embodiment, the heating zone 111 comprises a recess 111 for receiving at least a portion of the article. In other embodiments, the heating zone 111 may be other than a recess, such as a shelf, a surface, or a projection, and may require mechanical mating with the article in order to co-operate with, or receive, the article. In this embodiment, the heating zone 111 is elongate, and is sized and shaped to receive the article. In this embodiment, the heating zone 111 accommodates the whole article. In other embodiments, the heating zone 111 may be dimensioned to receive only a portion of the article.

In this embodiment, the magnetic field generator 112 comprises an electrical power source 113, a coil 114, a device 116 for passing a varying electrical current, such as an alternating current, through the coil 114, a controller 117, and a user interface 118 for user-operation of the controller 117.

In this embodiment, the electrical power source 113 is a rechargeable battery. In other embodiments, the electrical power source 113 may be other than a rechargeable battery, such as a non-rechargeable battery, a capacitor, a battery-capacitor hybrid, or a connection to a mains electricity supply.

The coil 114 may take any suitable form. In this embodiment, the coil 114 is a helical coil of electrically-conductive material, such as copper. In some embodiments, the magnetic field generator 112 may comprise a magnetically permeable core around which the coil 114 is wound. Such a magnetically permeable core concentrates the magnetic flux produced by the coil 114 in use and makes a more powerful magnetic field. The magnetically permeable core may be made of iron, for example. In some embodiments, the magnetically permeable core may extend only partially along the length of the coil 114, so as to concentrate the magnetic flux only in certain regions.

In this embodiment, the coil 114 is in a fixed position relative to the heater 115 and the heating zone 111. In this embodiment, the coil 114 encircles the heater 115 and the heating zone 111. In this embodiment, the coil 114 extends along a longitudinal axis that is substantially aligned with a longitudinal axis A-A of the heating zone 111. In this embodiment, the aligned axes are coincident. In a variation to this embodiment, the aligned axes may be parallel to each other. However, in other embodiments, the axes may be oblique to each other. Moreover, in this embodiment, the coil 114 extends along a longitudinal axis that is substantially coincident with a longitudinal axis of the heater 115. This can help to provide more uniform heating of the heater 115 in use, and can also aid manufacturability of the apparatus 100. In other embodiments, the longitudinal axes of the coil 114 and the heater 115 may be aligned with each other by being parallel to each other, or may be oblique to each other.

In this embodiment, the device 116 for passing a varying current through the coil 114 is electrically connected between the electrical power source 113 and the coil 114. In this embodiment, the controller 117 also is electrically connected to the electrical power source 113, and is communicatively connected to the device 116 to control the device 116. More specifically, in this embodiment, the controller 117 is for controlling the device 116, so as to control the supply of electrical power from the electrical power source 113 to the coil 114. In this embodiment, the controller 117 comprises an integrated circuit (IC), such as an IC on a printed circuit board (PCB). In other embodiments, the controller 117 may take a different form. In some embodiments, the apparatus may have a single electrical or electronic component comprising the device 116 and the controller 117. The controller 117 is operated in this embodiment by user-operation of the user interface 118. In this embodiment, the user interface 118 is located at the exterior of the body 110. The user interface 118 may comprise a push-button, a toggle switch, a dial, a touchscreen, or the like. In other embodiments, the user interface 118 may be remote and connected to the rest of the apparatus wirelessly, such as via Bluetooth.

In this embodiment, operation of the user interface 118 by a user causes the controller 117 to cause the device 116 to cause an alternating electrical current to pass through the coil 114, so as to cause the coil 114 to generate an alternating magnetic field. The coil 114 and the heater 115 of the apparatus 100 are suitably relatively positioned so that the alternating magnetic field produced by the coil 114 penetrates the heating material of the heater 115. When the heating material of the heater 115 is an electrically-conductive material, this may cause the generation of one or more eddy currents in the heating material. The flow of eddy currents in the heating material against the electrical resistance of the heating material causes the heating material to be heated by Joule heating. In this embodiment, the heating material is made of a magnetic material, and so the orientation of magnetic dipoles in the heating material changes with the changing applied magnetic field, which causes heat to be generated in the heating material.

A maximum temperature to which the heater 115 of the apparatus 100 is heatable by penetration with the varying magnetic field in use is exclusively determined by a Curie point temperature of the heating material of the heater 115. That is, the apparatus 100 may be free of any other system for limiting the temperature to which the heater 115 is heatable to below the maximum temperature. In this embodiment, the chemical composition of the heating material of the heater 115 of the apparatus 100 has been carefully and intentionally set, selected or provided so that the heating material has a Curie point temperature that is less than the combustion temperature of the smokable material in an article to be used with the apparatus 100. Accordingly, when the temperature of the heater 115 in use reaches the Curie point temperature, the ability to further heat the heater 115 by penetration with a varying magnetic field is reduced or removed, as discussed above.

Thus, in use, this inherent mechanism of the heating material of the heater 115 may be used to limit or prevent further heating of the heater 115, so as to help avoid the temperature of the heating zone 111 and an article located therein from reaching a magnitude at which the smokable material of the article burns or combusts. Thus, in some embodiments, the chemical composition of the heater 115 may help enable the smokable material to be heated sufficiently to volatilise at least one component of the smokable material without burning the smokable material. In some embodiments, this may also help to prevent overheating of the apparatus 100 or damage to components of the apparatus, such as the magnetic field generator 112.

As noted above, in some embodiments, the ability of the heating material to be heated by penetration with a varying magnetic field by magnetic hysteresis heating may return when the temperature of the heating material has dropped below the Curie point temperature.

In some embodiments, if the combustion temperature of the smokable material to be used with the apparatus 100 is greater than X degrees Celsius, then the chemical composition of the heating material may be provided so that the Curie point temperature is no more than X degrees Celsius. For example, if the combustion temperature of the smokable material is greater than 350 degrees Celsius, then the chemical composition of the heating material may be provided so that the Curie point temperature is no more than 350 degrees Celsius. The Curie point temperature may be, for example, less than 350 degrees Celsius, less than 325 degrees Celsius, less than 300 degrees Celsius, less than 280 degrees Celsius, less than 260 degrees Celsius, less than 240 degrees Celsius, or less than 220 degrees Celsius.

In some embodiments, the heater 115 may consist entirely, or substantially entirely, of the heating material. The heating material may comprise, for example, one or more materials selected from the group consisting of: iron; an alloy comprising iron; an alloy comprising iron and nickel; an alloy comprising iron and nickel and chromium; an alloy comprising iron and nickel and chromium and manganese; an alloy comprising iron and nickel and chromium and manganese and silicon; and stainless steel.

The apparatus 100 may comprise more than one coil. The plurality of coils of the apparatus 100 could be operable to provide progressive heating of the smokable material 10 in an article 1, and thereby progressive generation of vapour. For example, one coil may be able to heat a first region of the heating material relatively quickly to initialise volatilisation of at least one component of the smokable material 10 and formation of a vapour in a first region of the smokable material 10. Another coil may be able to heat a second region of the heating material relatively slowly to initialise volatilisation of at least one component of the smokable material 10 and formation of a vapour in a second region of the smokable material 10. Accordingly, a vapour is able to be formed relatively rapidly for inhalation by a user, and vapour can continue to be formed thereafter for subsequent inhalation by the user even after the first region of the smokable material 10 may have ceased generating vapour. The initially-unheated second region of smokable material 10 could act as a heat sink, to reduce the temperature of created vapour or make the created vapour mild, during heating of the first region of smokable material 10.

In some embodiments, the apparatus 100 may have a sensor for detecting a Curie-related change in magnetism of the heater 20, 115. The sensor may be communicatively-connected to the controller 117. The controller 117 may be configured to control the device 116 to cause the generation of the varying magnetic field to be halted or changed, on the basis of a signal received at the controller 117 from the sensor.

In some embodiments, the apparatus 100 may have an amplifier for amplifying the Curie-related change in magnetism of the heater 20, 115 of the article 1 or apparatus 100. For example, the coil 114 may be configured or arranged so that a change in a property of the coil 114 in response to the Curie-related change in magnetism of the heater 20, 115 is large. The impedance of the coil 114 may be matched with the impedance of the heater 20, 115, to result in a Curie-related event being more reliably detectable.

Referring to Figure 5, there is shown a flow diagram showing an example of a method of manufacturing a product for use in heating smokable material to volatilise at least one component of the smokable material, according to an embodiment of the invention. The apparatus 100 of Figure 3 may be made according to this method.

The method 500 comprises determining 501 a maximum temperature to which a heater is to be heated in use. The determining 501 may comprise, for example, determining the combustion temperature of smokable material to be heated by the heater 115 in use, and then determining the maximum temperature on the basis of that combustion temperature. For example, in some embodiments, the maximum temperature may be less than the combustion temperature of the smokable material, for the reasons discussed above. In other embodiments, the step of determining 501 may additionally or alternatively comprise determining a maximum comfortable temperature to which the exterior of the apparatus 100 is to be permitted to reach in use while still being comfortable to hold by a user, and then determining the maximum temperature on the basis of that temperature. In still further embodiments, the step of determining 501 may additionally or alternatively comprise determining a maximum temperature to which components, such as electrical components, of the apparatus 100 may be subjected in use without incurring damage, and then determining the maximum temperature on the basis of that temperature.

The method further comprises providing 502 a heater 115 comprising heating material, wherein the heating material is heatable by penetration with a varying magnetic field, and wherein the heating material has a Curie point temperature selected or determined on the basis of, or in dependence on, the maximum temperature determined at 501. The step of providing 502 may comprise, for example, manufacturing the heater 115 from suitable heating material. The method may comprise adjusting the composition of the heating material during manufacture of the heater 115. Alternatively or additionally, the step of providing 502 may comprise selecting the heater 115 from a plurality of heaters 115, wherein the plurality of heaters 115 are made of heating material having respective different Curie point temperatures.

The Curie point temperature of the heating material of the heater 115 provided in step 502 may, for example, be equal to the maximum temperature determined in step 501, or may be less than the maximum temperature determined in step 501. The heater 115 provided in step 502 may consists entirely, or substantially entirely, of the heating material. The heating material may comprise or consist of any one or more of the available heating materials discussed above, for example.

The method then comprises forming 503 apparatus, such as the apparatus 100 of Figure 3, that comprises a heating zone 111 for receiving an article comprising smokable material, the heater 115 for heating the heating zone 111, and a magnetic field generator 112 for generating a varying magnetic field that penetrates the heating material, wherein a maximum temperature to which the heater 115 is heatable by penetration with the varying magnetic field in use is exclusively determined by the Curie point temperature of the heating material.

In some examples, step 403 of the method 400 of Figure 4, and/or step 503 of the method 500 of Figure 5, may be omitted. For example, in some such examples, the product made using the method may be a component or system for future incorporation into apparatus for heating smokable material to volatilise at least one component of the smokable material. In some other such examples, the product made using the method may be a component or system for future incorporation into an article for use with such apparatus.

Accordingly, in accordance with embodiments of the present invention, a product, such as the apparatus 100 of Figure 3, may be provided with an automatic mechanism for limiting the temperature to which a heater 115 of the product is heatable by penetration with a varying magnetic field.

In each of the embodiments discussed above, the heating material may have a skin depth, which is an exterior zone within which most of an induced electrical current and/or induced reorientation of magnetic dipoles occurs. By providing that the component comprising the heating material has a relatively small thickness, a greater proportion of the heating material may be heatable by a given varying magnetic field, as compared to heating material in a component having a depth or thickness that is relatively large as compared to the other dimensions of the component. Thus, a more efficient use of material is achieved. In turn, costs are reduced.

In some embodiments, a component comprising the heating material may comprise discontinuities or holes therein. Such discontinuities or holes may act as thermal breaks to control the degree to which different regions of the smokable material 10 are heated in use. Areas of the heating material with discontinuities or holes therein may be heated to a lesser extent that areas without discontinuities or holes. This may help progressive heating of the smokable material 10, and thus progressive generation of vapour, to be achieved. Such discontinuities or holes may, on the other hand, be used to optimise the creation of complex eddy currents in use.

In each of the above described embodiments, the smokable material comprises tobacco. However, in respective variations to each of these embodiments, the smokable material may consist of tobacco, may consist substantially entirely of tobacco, may comprise tobacco and smokable material other than tobacco, may comprise smokable material other than tobacco, or may be free of tobacco. In some embodiments, the smokable material may comprise a vapour or aerosol forming agent or a humectant, such as glycerol, propylene glycol, triacetin, or diethylene glycol.

In each of the above described embodiments, the article is a consumable article. Once all, or substantially all, of the volatilisable component(s) of the smokable material in the article has/have been spent, the user may remove the article from the apparatus and dispose of the article. The user may subsequently re-use the apparatus with another of the articles. However, in other respective embodiments, the article may be non-consumable, and the apparatus and the article may be disposed of together once the volatilisable component(s) of the smokable material has/have been spent.

In some embodiments, the apparatus 100 discussed above is sold, supplied or otherwise provided separately from the articles with which the apparatus 100 is usable. However, in some embodiments, the apparatus 100 and one or more of the articles may be provided together as a system. Such systems may be in the form of a kit or an assembly, possibly with additional components, such as cleaning utensils.

Some of the products discussed herein may be considered smoking industry products.

## Claims

1. A method (500) of manufacturing a product comprising a heater for use in heating smokable material to volatilise at least one component of the smokable material, the method comprising:
determining (501) a maximum temperature to which a heater (115) is to be heated in use; and
providing (502) a heater (115) comprising heating material, wherein the heating material is heatable by penetration with a varying magnetic field, and wherein the heating material has a Curie point temperature selected on the basis of the determined maximum temperature; **characterised in that** the method comprises forming (503) apparatus for heating smokable material to volatilise at least one component of the smokable material, the apparatus comprising a heating zone (111) for receiving an article comprising smokable material, the heater (115) for heating the heating zone, and a magnetic field generator (112) for generating a varying magnetic field that penetrates the heating material; and
wherein a maximum temperature to which the heater is heatable by penetration with the varying magnetic field in use is exclusively determined by the Curie point temperature of the heating material.

2. The method of claim 1, wherein the Curie point temperature is equal to or less than the maximum temperature.

3. The method of claim 1 or claim 2, wherein the maximum temperature is less than the combustion temperature of the smokable material to be heated by the heater in use.

4. The method of any one of claims 1 to 3, wherein the Curie point temperature is no more than 350 degrees Celsius.

5. The method of any one of claims 1 to 4, wherein the Curie point temperature is less than 300 degrees Celsius.

6. The method of any one of claims 1 to 5, wherein the heating material comprises one or more materials selected from the group consisting of: iron; an alloy comprising iron; an alloy comprising iron and nickel; an alloy comprising iron and nickel and chromium; an alloy comprising iron and nickel and chromium and manganese; an alloy comprising iron and nickel and chromium and manganese and silicon; and stainless steel.

7. The method of any one of claims 1 to 6, wherein the heater consists entirely, or substantially entirely, of the heating material.

8. Apparatus (100) for heating smokable material to volatilise at least one component of the smokable material, the apparatus comprising:
a heating zone (111) for receiving an article comprising smokable material;
a heater (115) for heating the heating zone, wherein the heater comprises heating material that is heatable by penetration with a varying magnetic field; and
a magnetic field generator (112) for generating a varying magnetic field that penetrates the heating material; **characterized in that** a maximum temperature to which the heater is heatable by penetration with the varying magnetic field in use is exclusively determined by a Curie point temperature of the heating material.

9. The apparatus of claim 8, wherein the Curie point temperature is no more than 350 degrees Celsius.

10. The apparatus of claim 8 or claim 9, wherein the Curie point temperature is less than 300 degrees Celsius.

11. The apparatus of any one of claims 8 to 10, wherein the heating material comprises one or more materials selected from the group consisting of: iron; an alloy comprising iron; an alloy comprising iron and nickel; an alloy comprising iron and nickel and chromium; an alloy comprising iron and nickel and chromium and manganese; an alloy comprising iron and nickel and chromium and manganese and silicon; and stainless steel.

12. The apparatus of any one of claims 8 to 11, wherein the heater consists entirely, or substantially entirely, of the heating material.

13. A system, comprising:
apparatus (100) for heating smokable material to volatilise at least one component of the smokable material; and
an article for use with the apparatus, wherein the article comprises smokable material;
wherein the apparatus comprises:
a heating zone (111) for receiving the article,
a heater (115) for heating the smokable material when the article is in the heating zone, wherein the heater is formed of heating material that is heatable by penetration with a varying magnetic field, and
a magnetic field generator (112) for generating a varying magnetic field that penetrates the heating material; **characterised in that** a maximum temperature to which the heater is heatable by penetration with the varying magnetic field in use is exclusively determined by a Curie point temperature of the heating material.

## Patentansprüche

1. Verfahren (500) zur Herstellung eines Produkts, umfassend ein Heizelement zur Verwendung bei der Erwärmung eines rauchbaren Materials zum Verflüchtigen mindestens einer Komponente des rauchbaren Materials, wobei das Verfahren das Folgende umfasst:
Bestimmen (501) einer Höchsttemperatur, auf die ein Heizelement (115) bei der Benutzung aufgeheizt werden soll; und
Bereitstellen (502) eines Heizelements (115), umfassend Heizmaterial, wobei das Heizmaterial durch Penetration mit einem variierenden Magnetfeld erhitzbar ist und wobei das Heizmaterial eine Curie-Temperatur aufweist, die auf Basis der bestimmten Höchsttemperatur ausgewählt wird;
**dadurch gekennzeichnet, dass** das Verfahren die Bildung (503) einer Vorrichtung zum Erwärmen von rauchbarem Material zum Verflüchtigen mindestens einer Komponente des rauchbaren Materials umfasst, wobei die Vorrichtung eine Heizzone (111) zur Aufnahme eines rauchbares Material umfassenden Artikels, das Heizelement (115) zum Aufheizen der Heizzone und einen Magnetfeldgenerator (112) zum Erzeugen eines variierenden Magnetfelds, das das Heizmaterial penetriert, umfasst; und
wobei eine Höchsttemperatur, auf die das Heizelement durch Penetration mit dem variierenden Magnetfeld bei der Benutzung erhitzbar ist, ausschließlich durch die Curie-Temperatur des Heizmaterials festgelegt wird.

2. Verfahren nach Anspruch 1, wobei die Curie-Temperatur gleich oder kleiner als die Höchsttemperatur ist.

3. Verfahren nach Anspruch 1 oder Anspruch 2, wobei die Höchsttemperatur niedriger als die Verbrennungstemperatur des rauchbaren Materials ist, das vom Heizelement bei der Benutzung erwärmt werden soll.

4. Verfahren nach einem der Ansprüche 1 bis 3, wobei die Curie-Temperatur nicht mehr als 350 Grad Celsius beträgt.

5. Verfahren nach einem der Ansprüche 1 bis 4, wobei die Curie-Temperatur weniger als 300 Grad Celsius beträgt.

6. Verfahren nach einem der Ansprüche 1 bis 5, wobei das Heizmaterial ein oder mehrere Materialien umfasst, die aus der folgenden Gruppe ausgewählt sind:
Eisen; eine eisenhaltige Legierung; eine Eisen und Nickel enthaltende Legierung; eine Eisen und Nickel und Chrom enthaltende Legierung; eine Eisen und Nickel und Chrom und Mangan enthaltende Legierung; eine Eisen und Nickel und Chrom und Mangan und Silicium enthaltende Legierung; und Edelstahl.

7. Verfahren nach einem der Ansprüche 1 bis 6, wobei das Heizelement vollständig oder im Wesentlichen vollständig aus dem Heizmaterial besteht.

8. Vorrichtung (100) zum Erwärmen von rauchbarem Material zum Verflüchtigen zumindest einer Komponente des rauchbaren Materials, wobei die Vorrichtung das Folgende umfasst:
eine Heizzone (111) zur Aufnahme eines rauchbares Material umfassenden Artikels;
ein Heizelement (115) zum Aufheizen der Heizzone, wobei das Heizelement Heizmaterial umfasst, das durch Penetration mit einem variierenden Magnetfeld erhitzbar ist; und
einen Magnetfeldgenerator (112) zum Erzeugen eines variierenden Magnetfelds, das das Heizmaterial penetriert;
**dadurch gekennzeichnet, dass** eine Höchsttemperatur, auf die das Heizelement durch Penetration mit dem variierenden Magnetfeld bei der Benutzung erhitzbar ist, ausschließlich durch eine Curie-Temperatur des Heizmaterials festgelegt wird.

9. Verfahren nach Anspruch 8, wobei die Curie-Temperatur nicht mehr als 350 Grad Celsius beträgt.

10. Verfahren nach Anspruch 8 oder Anspruch 9, wobei die Curie-Temperatur weniger als 300 Grad Celsius beträgt.

11. Verfahren nach einem der Ansprüche 8 bis 10, wobei das Heizmaterial ein oder mehrere Materialien umfasst, die aus der folgenden Gruppe ausgewählt sind:
Eisen; eine eisenhaltige Legierung; eine Eisen und Nickel enthaltende Legierung; eine Eisen und Nickel und Chrom enthaltende Legierung; eine Eisen und Nickel und Chrom und Mangan enthaltende Legierung; eine Eisen und Nickel und Chrom und Mangan und Silicium enthaltende Legierung; und Edelstahl.

12. Verfahren nach einem der Ansprüche 8 bis 11, wobei das Heizelement vollständig oder im Wesentlichen vollständig aus dem Heizmaterial besteht.

13. System, umfassend:
Vorrichtung (100) zum Erwärmen von rauchbarem Material zum Verflüchtigen zumindest einer Komponente des rauchbaren Materials; und
einen Artikel zur Verwendung mit der Vorrichtung, wobei der Artikel rauchbares Material umfasst;
wobei die Vorrichtung das Folgende umfasst:
eine Heizzone (111) zur Aufnahme des Artikels,
ein Heizelement (115) zum Erwärmen des rauchbaren Materials, wenn sich der Artikel in der Heizzone befindet, wobei das Heizelement aus Heizmaterial besteht, das durch Penetration mit einem variierenden Magnetfeld erhitzbar ist, und
einen Magnetfeldgenerator (112) zum Erzeugen eines variierenden Magnetfelds, das das Heizmaterial penetriert;
**dadurch gekennzeichnet, dass** eine Höchsttemperatur, auf die das Heizelement durch Penetration mit dem variierenden Magnetfeld bei der Benutzung erhitzbar ist, ausschließlich durch eine Curie-Temperatur des Heizmaterials festgelegt wird.

## Revendications

1. Procédé (500) de fabrication d'un produit comprenant un dispositif de chauffage destiné à être utilisé en chauffage de matière pouvant être fumée pour volatiliser au moins un composant de la matière pouvant être fumée, le procédé comprenant :
la détermination (501) d'une température maximale à laquelle un dispositif de chauffage (115) doit être chauffé lors de l'utilisation ; et
l'utilisation (502) d'un dispositif de chauffage (115) comprenant du matériau chauffant, le matériau chauffant pouvant être chauffé par pénétration d'un champ magnétique variable et le matériau chauffant ayant une température de point de Curie choisie sur la base de la température maximale déterminée ;
**caractérisé en ce que**
le procédé comprend la formation (503) d'un appareil pour le chauffage de matière pouvant être fumée pour volatiliser au moins un composant de la matière pouvant être fumée, l'appareil comprenant une zone de chauffage (111) pour la réception d'un article comprenant de la matière pouvant être fumée, le dispositif de chauffage (115) pour le chauffage de la zone de chauffage et un générateur de champ magnétique (112) pour la production d'un champ magnétique variable qui pénètre dans le matériau chauffant ; et
dans lequel une température maximale à laquelle le dispositif de chauffage peut être chauffé par pénétration du champ magnétique variable lors de l'utilisation est exclusivement déterminée par la température du point de Curie du matériau chauffant.

2. Procédé selon la revendication 1, dans lequel la température du point de Curie est inférieure ou égale à la température maximale.

3. Procédé selon la revendication 1 ou la revendication 2, dans lequel la température maximale est inférieure à la température de combustion de la matière pouvant être fumée devant être chauffée par le dispositif de chauffage lors de l'utilisation.

4. Procédé selon l'une quelconque des revendications 1 à 3, dans lequel la température du point de Curie est inférieure ou égale à 350 degrés Celsius.

5. Procédé selon l'une quelconque des revendications 1 à 4, dans lequel la température du point de Curie est inférieure à 300 degrés Celsius.

6. Procédé selon l'une quelconque des revendications 1 à 5, dans lequel le matériau chauffant comprend un ou plusieurs matériaux choisis dans le groupe constitué par :
le fer ; un alliage comprenant du fer ; un alliage comprenant du fer et du nickel ; un alliage comprenant du fer et du nickel et du chrome ; un alliage comprenant du fer et du nickel et du chrome et du manganèse ; un alliage comprenant du fer et du nickel et du chrome et du manganèse et du silicium ; et l'acier inoxydable.

7. Procédé selon l'une quelconque des revendications 1 à 6, dans lequel le dispositif de chauffage est constitué entièrement, ou pratiquement entièrement, du matériau chauffant.

8. Appareil (100) pour le chauffage de matière pouvant être fumée pour volatiliser au moins un composant de la matière pouvant être fumée, l'appareil comprenant :
une zone de chauffage (111) pour la réception d'un article comprenant de la matière pouvant être fumée ;
un dispositif de chauffage (115) pour le chauffage de la zone de chauffage, le dispositif de chauffage comprenant du matériau chauffant qui peut être chauffé par pénétration d'un champ magnétique variable ; et
un générateur de champ magnétique (112) pour la production d'un champ magnétique variable qui pénètre dans le matériau chauffant ;
**caractérisé en ce que**
une température maximale à laquelle le dispositif de chauffage peut être chauffé par pénétration du champ magnétique variable lors de l'utilisation est exclusivement déterminée par une température de point de Curie du matériau chauffant.

9. Appareil selon la revendication 8, dans lequel la température du point de Curie est inférieure ou égale à 350 degrés Celsius.

10. Appareil selon la revendication 8 ou la revendication 9, dans lequel la température du point de Curie est inférieure à 300 degrés Celsius.

11. Appareil selon l'une quelconque des revendications 8 à 10, dans lequel le matériau chauffant comprend un ou plusieurs matériaux choisis dans le groupe constitué par :
le fer ; un alliage comprenant du fer ; un alliage comprenant du fer et du nickel ; un alliage comprenant du fer et du nickel et du chrome ; un alliage comprenant du fer et du nickel et du chrome et du manganèse ; un alliage comprenant du fer et du nickel et du chrome et du manganèse et du silicium ; et l'acier inoxydable.

12. Appareil selon l'une quelconque des revendications 8 à 11, dans lequel le dispositif de chauffage est constitué entièrement, ou pratiquement entièrement, du matériau chauffant.

13. Système, comprenant :
un appareil (100) pour le chauffage de matière pouvant être fumée pour volatiliser au moins un composant de la matière pouvant être fumée ; et
un article destiné à être utilisé avec l'appareil, l'article comprenant de la matière pouvant être fumée ;
dans lequel l'appareil comprend :
une zone de chauffage (111) pour la réception de l'article,
un dispositif de chauffage (115) pour le chauffage de la matière pouvant être fumée lorsque l'article est dans la zone de chauffage, le dispositif de chauffage étant formé de matériau chauffant qui peut être chauffé par pénétration d'un champ magnétique variable, et
un générateur de champ magnétique (112) pour la production d'un champ magnétique variable qui pénètre dans le matériau chauffant ;
**caractérisé en ce que**
une température maximale à laquelle le dispositif de chauffage peut être chauffé par pénétration du champ magnétique variable lors de l'utilisation est exclusivement déterminée par une température de point de Curie du matériau chauffant.
